# EUROPEAN PATENT APPLICATION

(11) **EP 3 135 279 A1**
(43) Date of publication of application: **01.03.2017**
(21) Application number: 15783638.8
(22) Date of filing: 30.03.2015
(51) Int. Cl.: A61K 31/194, A61K 9/48, A61K 47/44, A61P 9/00, A61P 25/28, A61P 37/08

(54) **UNSATURATED FATTY ACID ABSORPTION ACCELERATOR**

(30) Priority: 25.04.2014 JP 2014091281
(71) Applicant: Yamada Bee Company, Inc., Tomata-gun, Okayama 708-0393 (JP)
(72) Inventor: WATANABE Nakamichi, Tokyo 154-8533 (JP); SADO Tetuya, Tomata-gun Okayam 708-0393 (JP); NAKATUKA Hiromi, Tomata-gun Okayam 708-0393 (JP); KONO Takayuki, Tomata-gun Okayam 708-0393 (JP)
(74) Representative: Enomoto, Kéi
(86) International application number: PCT/JP2015/059907
(87) International publication number: WO 2015/163091

(57) **Abstract**

The present invention provides an unsaturated fatty acid absorption accelerator comprising a wax as an active ingredient. The absorption of unsaturated fatty acids can be accelerated by using the absorption accelerator of the present invention.

## Description

### Technical Field

The present invention relates to an unsaturated fatty acid absorption accelerator.

### Background Art

Beeswax is a product obtained by refining a honeycomb, as a raw material. A honeycomb is composed of a wax secreted from the wax-producing glands of honey bees (Non Patent Literature 1). The beeswax is used for the purpose of cosmetics and candles and the like. The beeswax is also used for the purpose of medical treatments and pharmaceuticals in the form of white beeswax (e.g., Patent Literature 1).

N-3 Polyunsaturated fatty acids, such as eicosapentaenoic acid (EPA), docosahexaenoic acid (DHA) and α-linolenic acid contained in fish oils, have been reported as having various physiological functions such as improvement effects on cardiovascular disease and blood fluidity, anti-allergic effects and preventive effects on cognitive impairment. N-6 Polyunsaturated fatty acids, such as arachidonic acid, have been reported as being essential for the development of the brain and body of infants and having effects to regulate the immune function and enhance the learning ability and memory. These unsaturated fatty acids can hardly be biosynthesized in the body and hence need to be ingested from outside the body. According to "Dietary Reference Intakes for Japanese (2010)" developed by the Ministry of Health, Labor and Welfare, an adult must ingest about 2 g of n-3 fatty acids a day and about 1 g of EPA and DHA a day. In recent years, a wide variety of supplements containing n-3 polyunsaturated fatty acids, such as EPA and DHA, are available.

However, n-3 polyunsaturated fatty acids, such as EPA and DHA, pose a problem of having a low absorption rate in the body. Studies have been conducted to enhance the absorption rate of these unsaturated fatty acids.

Patent Literature 2, for example, discloses the use of lecithin in a nutrition enforced food product which contains an edible oil containing docosahexaenoic acid, eicosapentaenoic acid, or a mixture thereof to improve the absorption of the edible oil in the body. Patent Literature 3 discloses a method for increasing a DHA concentration in blood by administrating a composition containing DHA and a mixture of phosphatidylcholine and lysophosphatidylcholine in a ratio of 90:10 to 10:90.

### Citation List

### Patent Literature

Patent Literature 1: JP S56-131514A
Patent Literature 2: JP S59-82070A
Patent Literature 3: JP2009-249354A

### Non Patent Literature

Non Patent Literature 1: "Yoho no Kagaku (in Japanese)" ("Science of Apiculture"), written by Masami Sasaki, Science House Inc. 1996.

### Summary of Invention

### Technical Problem

Most of the typical conventional absorption accelerators do not meet the need of consumers demanding the use of raw materials of natural origin. Lecithin used in Patent Literature 2 is believed to cause diarrhea, nausea, stomach ache and/or obesity as side effects when orally ingested (National Institute of Health and Nutrition, an independent administrative corporation. http://hfnet.nih.go.jp/contents/detail53.html). Phosphatidylcholine used in Patent Literature 3 raises a problem of high production costs thereof. Thus, the choice of ingredients capable of accelerating the absorption of unsaturated fatty acids has been limited up to date and other substitutes which replace therewith have been sought for.

The present invention has an object to provide an absorption accelerator capable of enhancing the absorption rate of unsaturated fatty acids.

### Solution to Problem

The present invention provides a unsaturated fatty acid absorption accelerator comprising a wax as the active ingredient.

The present invention has been accomplished based on the finding, which has been found for the first time by the present inventors, that the wax has an effect to accelerate the absorption of unsaturated fatty acids. The absorption accelerator of the present invention can accelerate the absorption of unsaturated fatty acids in the body by the use of a wax.

It is preferable for the wax to be a beeswax. The beeswax is an ingredient of natural origin, safer than phosphatidylcholine, and available at a lower price. Consequently when such a beeswax is used, an absorption accelerator which can meet the recent consumers' need of natural consciousness and is hence easily accepted by many more consumers can be provided.

In the absorption accelerator, it is preferable that the unsaturated fatty acid be one or more selected from the group consisting of n-3 polyunsaturated fatty acids and n-6 polyunsaturated fatty acids. When the above absorption accelerator is used as the absorption accelerator for one or more selected from the group consisting of n-3 polyunsaturated fatty acids and n-6 polyunsaturated fatty acids, a much higher absorption accelerating effect can be achieved. It is further preferable that the unsaturated fatty acid be at least one selected from the group consisting of eicosapentaenoic acid and docosahexaenoic acid. When the above absorption accelerator is used as the absorption accelerator for at least one selected from the group consisting of eicosapentaenoic acid and docosahexaenoic acid, a much higher absorption accelerating effect can be achieved.

The present invention also provides a capsule comprising a capsule film and a composition encapsulated inside the capsule film, wherein the composition comprises a wax and a unsaturated fatty acid. The capsule may alternatively comprise a capsule film and a composition encapsulated inside the capsule film, wherein the capsule film comprises a wax and wherein the composition comprises an unsaturated fatty acid. The capsule of the present invention enables the ingestion of a wax and an unsaturated fatty acid in combination, whereby the absorption of the unsaturated fatty acid in the body can be efficiently accelerated.

In the capsule, it is preferable that the unsaturated fatty acid be one or more selected from the group consisting of n-3 polyunsaturated fatty acids and n-6 polyunsaturated fatty acids. The capsule can more efficiently accelerate the absorption when one or more unsaturated fatty acids selected from the group consisting of n-3 polyunsaturated fatty acids and n-6 polyunsaturated fatty acids are used. It is further preferable that the unsaturated fatty acid be at least one selected from the group consisting of eicosapentaenoic acid and docosahexaenoic acid. In this way, a much higher absorption accelerating effect can be achieved.

### Advantageous Effects of Invention

The absorption of unsaturated fatty acids can be accelerated by using the absorption accelerator of the present invention. According to the capsule of the present invention, unsaturated fatty acids can be absorbed with a high efficiency.

### Brief Description of Drawings

Figure 1(a) is a graph showing over-time changes in the concentrations of EPA in the plasma and Figure 1(b) is a graph showing over-time changes in the concentrations of DHA in the plasma.
Figure 2(a) is a graph showing over-time changes in the concentrations of (a) DPA in the plasma and Figure 2(b) is a graph showing over-time changes in the concentrations of palmitoleic acid in the plasma.
Figure 3(a) is a graph showing over-time changes in the concentrations of α-linolenic acid in the plasma and Figure 3(b) is a graph showing over-time changes in the concentrations of oleic acid in the plasma.
Figure 4 is a graph showing over-time changes in the concentrations of arachidonic acid in the plasma.
Figure 5(a) is a graph showing over-time changes in the concentrations of EPA in the lymph fluid and Figure 5(b) is a graph showing over-time changes in the concentrations of DHA in the lymph fluid.
Figure 6 is a graph showing over-time changes in the concentrations of EPA and DHA in the plasma.

### Description of Embodiments

Hereinafter, the embodiments to carry out the present invention are described in detail. However, the present invention is not limited to the following embodiments.

The unsaturated fatty acid absorption accelerator of the present invention comprises a wax as the active ingredient.

Any wax can be used without limitation as long as it is ingestible. It is preferable for the wax to be those of natural origin. The waxes usable include, for example, those listed in "List of Existing Food Additives" compiled by The Japan Food Chemical Research Foundation, a public interest incorporated foundation. Specific examples include beeswax, urushi wax, carnauba wax, candelilla wax, rice bran wax, cane wax, shellac wax, jojoba wax, Japan wax and lanolin. The wax may be used alone, or two or more may be used in combination. Of these waxes, beeswax is particularly preferable due to a high effect to accelerate the absorption of unsaturated fatty acids.

Beeswax is a product obtained by refining, as a raw material, a honeycomb (a hive of worker bee) mainly composed of a wax secreted from the wax-producing glands of honey bees. The main component of beeswax is the wax secreted from the wax-producing glands of honey bees but also includes a part of pollen and propolis and the like adhered to the honeycomb. Any known refining method can be used without limitation and examples include the heat method utilizing solar heat and the hot press method.

The honeycomb to be the raw material of beeswax is not particularly limited and examples may include the honeycomb of European honey bee (*Apis mellifera Linne*), African honey bees (*Apis mellifera scutellata* and *Apis mellifera adansonii*), Eastern honey bee (*Apis cerana Fabricius* (*Apidae*)), giant honey bee (*Apis dorsata*) and small honey bee (*Apis florea*). Of these, the honeycombs of European honey bees and African honey bees are preferable due to a high absorption accelerating effect of unsaturated fatty acids and easy availability. The melting point of beeswax is, for example, 60 to 67°C, and may be 62 to 65°C.

The beeswax may be, for example, yellow beeswax or white beeswax as defined in The Japanese Pharmacopoeia Sixteenth Edition. White beeswax is a product obtained by bleaching the yellow beeswax. Yellow beeswax is preferably used particularly by the reason of easy production.

The absorption accelerator according to the present embodiment may consist only of the wax which is the active ingredient or may contain other ingredients as long as the absorption accelerating effect of the wax is not inhibited. Examples of the other ingredients include pharmaceutically acceptable additives (excipients, binders, lubricants, disintegrators, emulsifiers, surfactants, bases, solubilizing adjuvants and suspension agents).

The absorption accelerator according to the present embodiment may be in any state of solids, liquids or pastes, and may be a form of tablets (including uncoated tablets, sugar-coated tablets, foaming tablets, film-coated tablets, chewable tablets and troches), capsules, pills, powders (fine powders), fine granules, granules, liquids, suspensions, emulsions, syrups, pastes or injections (including the case where a liquid is prepared by adding to distilled water or a transfusion solution such as an amino acid transfusion solution or electrolyte transfusion solution). Each of these preparations can be prepared by, for example, mixing the wax and other ingredients and molding the mixture into the above dosage forms.

The percentage of the wax contained in the absorption accelerator according to the present embodiment is not particularly limited as long as it is the range within which the effect of the present invention is achieved and can be suitably adjusted in accordance with the final form, with an example of a range from 0.01 to 100 mass% in total to suitably select from. It is particularly preferable to be in a range from 0.5 to 10 mass%.

Unsaturated fatty acids are referred to fatty acids which have a carbon-carbon double bond (an unsaturated bond) in a molecule. The unsaturated fatty acid may contain one unsaturated bond in a molecule or may be a highly unsaturated fatty acid which contains a plurality of unsaturated bonds. Examples of the highly unsaturated fatty acid include those containing 2 to 7 unsaturated bonds in a molecule, with those containing 5 to 7 unsaturated bonds in a molecule being preferable. The number of carbon atoms in the unsaturated fatty acid is, for example, 10 or more, and may be 16 to 24. It is preferable that the unsaturated fatty acid have all of the double bonds contained in molecules in the cis arrangement. The unsaturated fatty acid may be a salt formed with an alkali metal, an alkaline earth metal, another metal or ammonium, or an amide, acceptable for use of food or medical treatment, or a lower alcohol ester such as methyl ester or ethyl ester, or other derivatives.

Examples of the highly unsaturated fatty acid include n-3 polyunsaturated fatty acids and n-6 polyunsaturated fatty acids, with n-3 polyunsaturated fatty acids being more preferable. The n-3 fatty acid refers to the unsaturated fatty acid having a carbon-carbon double bond at the ω-3 position. When the absorption accelerator according to the present embodiment is used to accelerate the absorption of n-3 polyunsaturated fatty acids, the absorption can further be efficiently accelerated. Examples of the n-3 polyunsaturated fatty acid include EPA, DHA, docosapentaenoic acid (DPA) and α-linolenic acid (ALA). Example of the n-6 polyunsaturated fatty acid include arachidonic acid, linoleic acid, γ-linolenic acid, eicosadienoic acid, dihomo-γ-linolenic acid, docosadienoic acid, docosatetraenoic acid and docosapentaenoic acid.

Examples of other unsaturated fatty acids include conjugated linoleic acid (CLA), α- or β-eleostearic acid, palmitoleic acid and oleic acid. The absorption accelerator according to the present embodiment may be used to accelerate the absorption of a mixture of two or more unsaturated fatty acids. It is preferable for the unsaturated fatty acid to be one or more selected from the group consisting of EPA, DHA, DPA, palmitoleic acid, ALA, oleic acid and arachidonic acid, more preferable to be one or more selected from the group consisting of EPA, DHA, DPA and palmitoleic acid, and particularly preferable to be at least one of EPA and DHA.

EPA is an unsaturated fatty acid represented by (5Z,8Z,11Z,14Z,17Z)-eicosa-5,8,11,14,17-pentaenoic acid and has five unsaturated bonds in the cis arrangement. DHA is the general term for fatty acids having six double bonds in a molecule or for the compounds having such a fatty acid as the structural component, and may be used in the form of a derivative thereof. For example, (4Z,7Z,10Z,13Z,16Z,19Z)-docosa-4,7,10,13,16,19-hexaene acid may be used. For DPA, 4Z,7Z,10Z,13Z,16Z-docosapentaenoic acid, which is n-3, or 7Z,10Z,13Z,16Z,19Z-docosapentaenoic acid, which is n-6, may be used. α-Linolenic acid is all-cis-9,12,15-octadecatrienoic acid.

The unsaturated fatty acid may be, for example, a structural component of a fatty acid salt, an ester formed with glycerol or ethanol, or phospholipid. The unsaturated fatty acid may also be a highly purified product or a composition containing such a product. The composition may be a naturally occurred composition such as fish oil or extracted oil of microalgae. Fish oil and extracted oil of microalgae of natural origin contain unsaturated fatty acids, such as EPA and DHA, in the form of triglyceride in a high content, hence preferable. It is particularly preferable for the unsaturated fatty acid to be an origin of fish oils.

The absorption accelerator according to the present embodiment may further comprise unsaturated fatty acids. When the absorption accelerator comprises unsaturated fatty acids, the wax and unsaturated fatty acids can be simultaneously ingested whereby the unsaturated fatty acids can be absorbed much easily with a higher efficiency.

The absorption accelerator according to the present embodiment can be used for an adult weighing 60 kg, on a wax content basis, for example, in a daily dose of 1 mg or more and 10 g or less, and it is preferable to use in a daily dose of 10 mg or more and 2 g or less, and more preferable to use in a daily dose of 20 mg or more and 100 mg or less. The dose can be suitably determined within the above ranges in accordance with factors such as health conditions of a person to ingest, administration method and the combination with other agents.

The absorption accelerator according to the present embodiment may be orally administered (ingested) or parenterally administered. The absorption accelerator according to the present embodiment can accelerate the absorption of unsaturated fatty acids and hence it is preferable to be orally administered or ingested as added to a drink or food. The absorption accelerator according to the present embodiment may be administered once a day or divided in multiple doses such as twice a day or three times a day as long as the daily active ingredient amount is within the above ranges. The absorption accelerator according to the present embodiment can be used for the purpose of food and pharmaceuticals.

The present invention can also be the use of a wax for accelerating the absorption of unsaturated fatty acids. The present invention can also be an application of a wax as the unsaturated fatty acid absorption accelerator, or an application of a wax for producing the unsaturated fatty acid absorption accelerator.

The present invention can further be a method for accelerating the absorption of unsaturated fatty acids comprising a step of administering a composition comprising a wax as the active ingredient (the unsaturated fatty acid absorption accelerator described above). The composition comprising a wax as the active ingredient and unsaturated fatty acids may be administered separately or may be administered together. The composition comprising a wax as the active ingredient may be administered to human or non-human mammals.

### (Food product composition)

The absorption accelerator according to the present embodiment can be used as a food product composition. The food product composition encompasses many food product compositions which are ingestible by animals (including human).

The food product composition can contain, as necessary minerals, vitamins, flavonoids, quinones, polyphenols, amino acids, nucleic acids, essential fatty acids, a refreshing agent, a binder, a sweetener, a disintegrator, a lubricant, a coloring agent, a flavor, a stabilizer, a preservative, a release adjusting agent, a surfactant, a solubilizer and/or a wetting agent.

The kind of food product composition is not particularly limited and examples include drinks (soft drinks such as coffees, juices and teas, milk-based drinks, lactic drinks, yogurt drinks, carbonated drinks, alcohols such as rice wines, liquors, fruit liquors and meads); spreads (custard creams); pastes (fruit pastes); pastries (chocolates, donuts, pies, cream puffs, gums, jellies, candies, cookies, cake and puddings); Japanese pastries (daifuku, mochi, manju, castella, anmitsu and yokan); frozen sweets (ice creams, popsicles and sorbets); food products (curries, gyudon, porridges, miso soups, soups, meat sauces, pastas, pickles, jams, honeys and propolis); and seasonings (dressings, sprinkles, flavor enhancing seasonings and soup mixes).

The method for producing the food product composition is not particularly limited and can suitably follow known methods. For example, the wax is mixed in the intermediate product or the final product during the production steps of the food product composition to obtain the food product composition to be used for the above object.

The food product composition can also be used as a health food product, a functional food product, a nutrition enforced food product, a supplement or a food for specified health use for accelerating the absorption of unsaturated fatty acids. The unit dosage form when used as a supplement can be suitably selected without being particularly limited and examples include tablets, capsules, granules, liquids and fine powders. Of these, capsules are preferable due to an increased absorption efficiency of unsaturated fatty acids, good storability, easy handleability and easy ingestability.

The pharmaceutical products or food products consisting of the absorption accelerator according to the present embodiment or the pharmaceutical products or food products comprising the absorption accelerator according to the present embodiment may be intended for accelerating the absorption of unsaturated fatty acids. Unsaturated fatty acids, such as DHA and EPA, have the effect to make the blood less viscous and the effect to enhance memory and concentration, and for this reason the pharmaceutical products or food products described above may be labeled, for example, with statements suggesting that the absorption of unsaturated fatty acids is accelerated, the blood becomes less viscous and memory and concentration are enhanced. The above pharmaceutical products or food products may also be labeled with statements suggesting that the products are suitable for those who are concerned about triglyceride; suitable for those with high cholesterol; beneficial to brain development; beneficial to atopic dermatitis and allergies; preventive of hypertension; lower blood glucose; preventive and beneficial to improvement of arteriosclerosis, hyperlipidemia, thrombosis and dementia; suppress incidence and metastasis of cancers; preventive of cardiovascular diseases; relieve symptoms of neuropathy and rheumatoid arthritis derived from diabetes; and regulate the immune function.

The capsule may comprise a capsule film and a composition encapsulated inside the capsule film, wherein the composition comprises the wax and a unsaturated fatty acid. Alternatively the capsule may comprise a capsule film and a composition encapsulated inside the capsule film, wherein the composition comprises a unsaturated fatty acid and wherein the capsule film comprises the wax. The capsule film is typically made of a gelatin. In addition, the shape of the capsule is not particularly limited and can be in any shape such as a round shape, a football shape or an elongated shape. The capsule may be, for example, a soft capsule or a hard capsule.

The composition in the above capsule is described. The content of unsaturated fatty acids in the composition is not particularly limited and can be, for example, 30 mass% to 99 mass%, and it is preferable to be 50 mass% to 99 mass%, with respect to the total amount of the composition. When the composition comprises the wax, the content of the wax is not particularly limited as long as it is the range within which the effect of the present invention can be achieved and can be, for example, 0.01 mass% to 99 mass%, and it is preferable to be 0.5 mass% to 10 mass%, with respect to the total amount of the composition.

The above composition may further comprise additional ingredients such as propolis, a gingko leaf extract, GABA, vitamin E. When these additional ingredients are contained, effects to inhibit the oxidation of unsaturated fatty acids can be expected. When additional ingredients are contained, the content of the additional ingredients can be, for example, 20 to 80 mass% on the basis of total amount of additional ingredients with respect to the total amount of the composition.

The wax may be contained in the capsule film. In such a case, the content of the wax in the capsule film is not particularly limited and it can be, for example, 0.01 mass% to 100 mass%, and it is preferable to be 1 mass% to 10 mass%, with respect to the total amount of the capsule film.

The above capsule may be those wherein the wax is contained in both of the composition and the capsule film. Note that preferable embodiments of an unsaturated fatty acid and the wax in the capsule are as described in the above absorption accelerator.

The above mentioned capsule comprises the wax and a unsaturated fatty acid integrally, and hence the ingestion of such a capsule enables the ingestion of unsaturated fatty acids easily with a high absorption efficiency.

In another embodiment of the present invention, the composition described above may be used directly without being encapsulated in the capsule film. In such a case, the composition can be obtained in accordance with the form thereof by the same method as in the case of producing the food product composition as described above or the pharmaceutical composition to be described later.

### (Pharmaceutical composition)

The absorption accelerator according to the present embodiment can be used in the form of pharmaceuticals (particularly oral pharmaceuticals).

For the preparation of the pharmaceutical composition, the wax, together with a nontoxic carrier, diluent or excipient acceptable in pharmaceutical products, is prepared into the forms such as tablets (including uncoated tablets, sugar-coated tablets, foaming tablets, film-coated tablets, chewable tablets and troches), capsules, pills, powders (fine powders), fine granules, granules, liquids, suspensions, emulsions, syrups, pastes or injections (including the case where a liquid is prepared by adding to distilled water or a transfusion solution such as an amino acid transfusion solution or electrolyte transfusion solution) to obtain pharmaceutical preparations. Preferable forms of the capsule are as described above.

### Examples

The present invention is described in more details in the following Examples. However, the present invention is not limited to the following Examples.

### [Test Example I]

The composition A obtained by mixing a refined fish oil and a beeswax in a mass ratio of 95:5 and the composition B obtained by mixing a refined fish oil and an olive oil in a mass ratio of 95:5 were prepared. "EPA45" of Miki Chemical Industry & Co., Ltd., as the refined fish oil, "MITSUROU Bee's Wax" of Miki Chemical Industry & Co., Ltd., as the beeswax and "Nisshin saratto karui (in Japanese) (flowable and light) Olive Oil" of The Nisshin Oillio Group, Ltd. as the olive oil were used. The concentrations of EPA and DHA in the refined fish oil used were about 47% and 16%, respectively.

### (Test Example I-1) Migration of the ingested unsaturated fatty acids to the blood

11 Week-old male DDY mice were fed with a commercial solid chow ("CRF-1" of Charles River Japan, Inc.) for one week of preliminary rearing and divided into two groups, followed by 16-hour fasting. After fasting, the composition A or the composition B was administered by gavage to each of the two groups using a sonde. The amount of the sample administered by gavage was 0.1 mL per body weight of 30 g. Dissection was conducted immediately after administration (0 hour later) and 1, 2, 3, 4, 5 and 6 hours after administration and blood was collected (n = 3). The collected blood was immediately centrifuged (1900 g, 10 min) to obtain the plasma. The plasma was stored at -30°C until analyzed.

### (Test Example I-2) Migration of the ingested EPA and DHA to the lymph fluid

11 Week-old male Wistar rats were fed with a commercial solid chow ("CRF-1" of Charles River Japan, Inc.) for one week of preliminary rearing and divided into two groups, followed by 16-hour fasting. After fasting, the composition A or the composition B was administered by gavage to each of the two groups using a sonde. The amount of the sample administered by gavage was 1 mL per body weight of 100 g. The lymph fluids were collected every hour up to 6 hours after administration. All the experiments on animals carried out in these Examples were practiced in conformity with ethics code for use of animals in research after approved by the experimental animal committee of Showa Women's University.

The total lipid was obtained by the Folch method from the plasma and the lymph fluid samples obtained in the above Test Examples I-1 and I-2. The obtained total lipid was methyl-esterified by the hydrochloric acid-methanol method and subsequently subjected to gas chromatography. The plasma samples were measured for the concentrations of EPA, DHA, DPA, palmitoleic acid, α-linolenic acid, oleic acid and arachidonic acid, whereas the lymph fluid samples were measured for the concentrations of EPA and DHA.

### (Result) Migration of the ingested unsaturated acids to the plasma

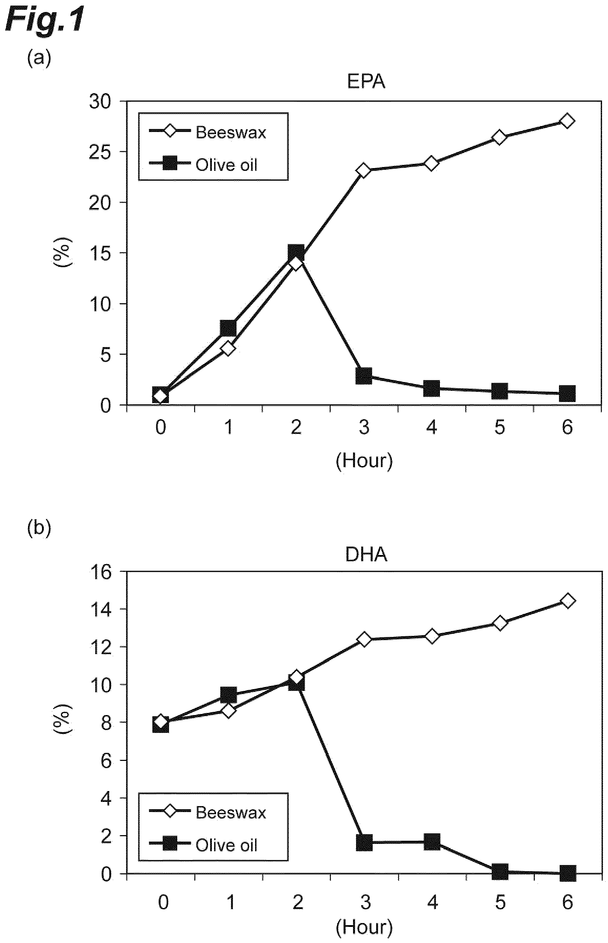

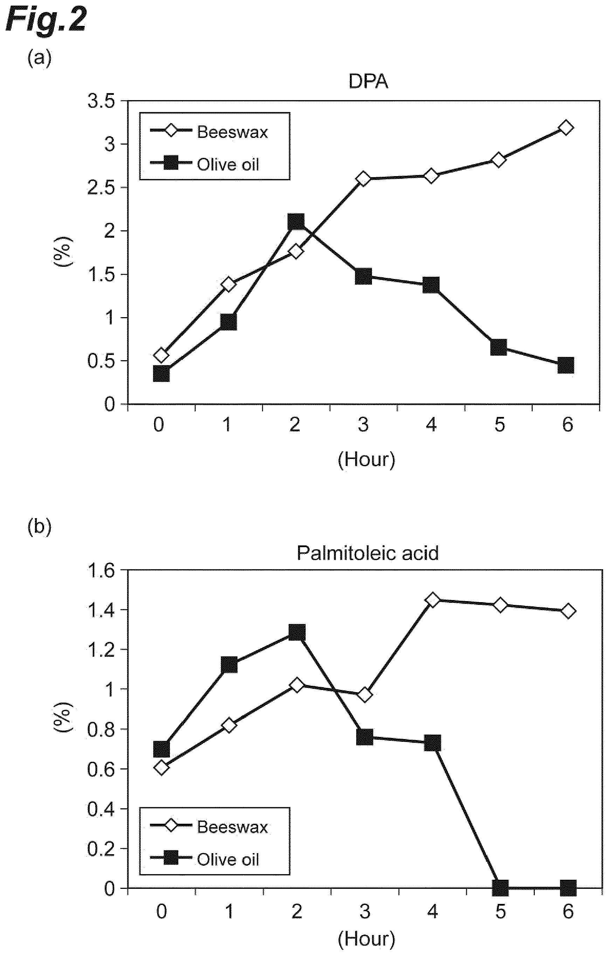

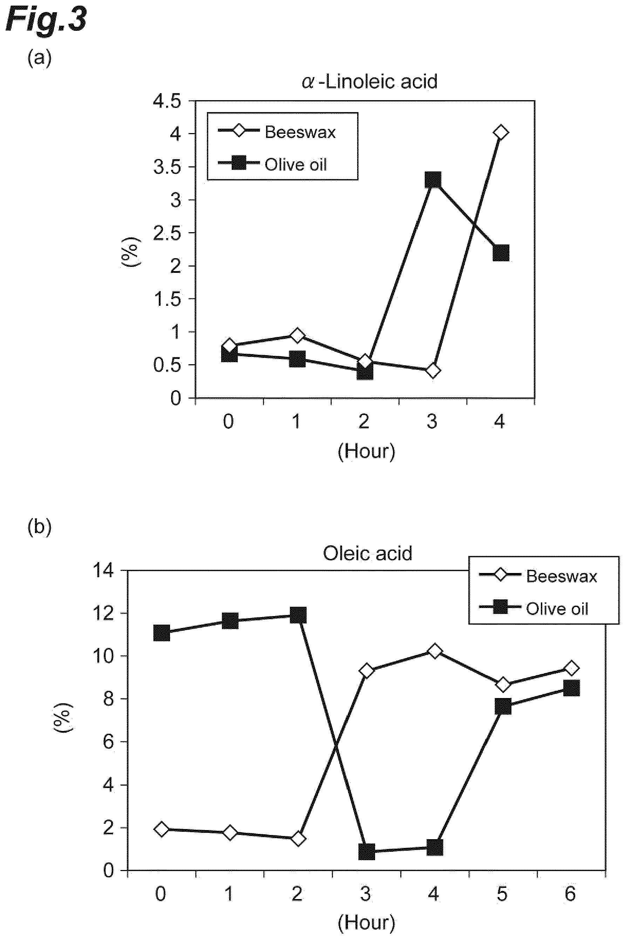

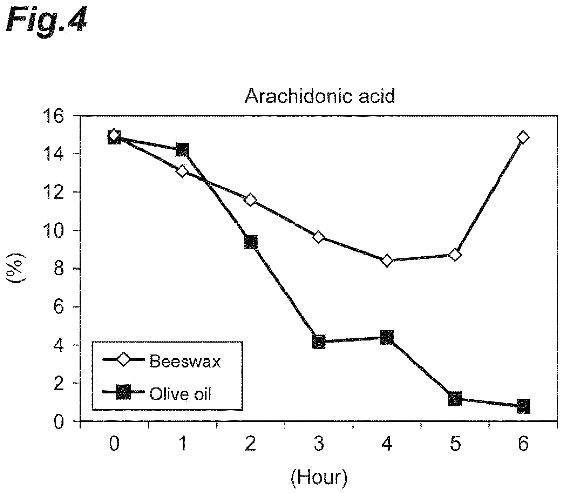

The EPA concentrations in the plasma samples 0, 1, 2, 3, 4, 5 and 6 hours after gavage administration of the mixtures of the refined fish oil and the beeswax or the olive oil are shown in Figures 1, 2 and 3. The EPA, DHA and DPA concentrations in the plasma of the group to which the olive oil was added all reached the peak 2 hours later (Figure 1 (a), Figure 1 (b), Figure 2 (a)). On the other hand, the EPA, DHA and DPA concentrations in the plasma of the group to which the beeswax was added continued increasing up to 6 hours after administration. The plasma palmitoleic acid concentration in the group to which the olive oil was added reached the peak about 2 hours later and then reduced, whereas the group to which the beeswax was added maintained high levels up to 6 hours later (Figure 2 (b)). All of these unsaturated fatty acids showed higher maximum concentrations in the case where the beeswax was added than the case where the olive oil was added. The plasma α-linolenic acid concentrations had higher concentration maximum values in the group to which the beeswax was added than the group to which the olive oil was added (Figure 3 (a)). The plasma oleic acid concentrations showed high values 3 hours later and thereafter in the group to which the beeswax was added, whereas the group to which the olive oil was added showed the reduction immediately after administration (Figure 3 (b)). The plasma arachidonic acid concentration was more increased in the group to which the beeswax was added than the group to which the olive oil was added (Figure 4). It is revealed that the absorption of these unsaturated fatty acids is accelerated by ingesting the unsaturated fatty acids with the beeswax.

Table 1 shows the increase rates of each of the unsaturated fatty acid concentrations in the plasma at each elapsed time when 0 hour after gavage administration of the sample is 100%. The concentrations in the plasma 6 hours after gavage administration were 3168% in EPA, 180% in DHA, 566% in DPA and 230% in palmitoleic acid.

**[Table 1]**

| | | 0 h | 1 h | 2 h | 3 h | 4 h | 5 h | 6 h |
|---|---|---|---|---|---|---|---|---|
| EPA | Beeswax | 100 | 630 | 1577 | 2615 | 2693 | 2984 | 3168 |
| | Olive oil | 100 | 741 | 1470 | 280 | 161 | 133 | 110 |
| DHA | Beeswax | 100 | 107 | 129 | 154 | 156 | 165 | 180 |
| | Olive oil | 100 | 120 | 128 | 21 | 21 | 1 | 0 |
| DPA | Beeswax | 100 | 245 | 313 | 461 | 467 | 499 | 566 |
| | Olive oil | 100 | 270 | 597 | 419 | 390 | 187 | 127 |
| Palmitoleic acid | Beeswax | 100 | 135 | 168 | 160 | 239 | 235 | 230 |
| | Olive oil | 100 | 161 | 184 | 109 | 105 | 0 | 0 |
| α-Linoleic acid | Beeswax | 100 | 120 | 69 | 52 | 511 | - | - |
| | Olive oil | 100 | 89 | 60 | 499 | 332 | - | - |
| Oleic acid | Beeswax | 100 | 92 | 77 | 483 | 532 | 450 | 490 |
| | Olive oil | 100 | 105 | 107 | 8 | 10 | 69 | 77 |

The unit for numerical values in Table is %.

### Migration of the ingested EAP and DHA to the lymph fluid

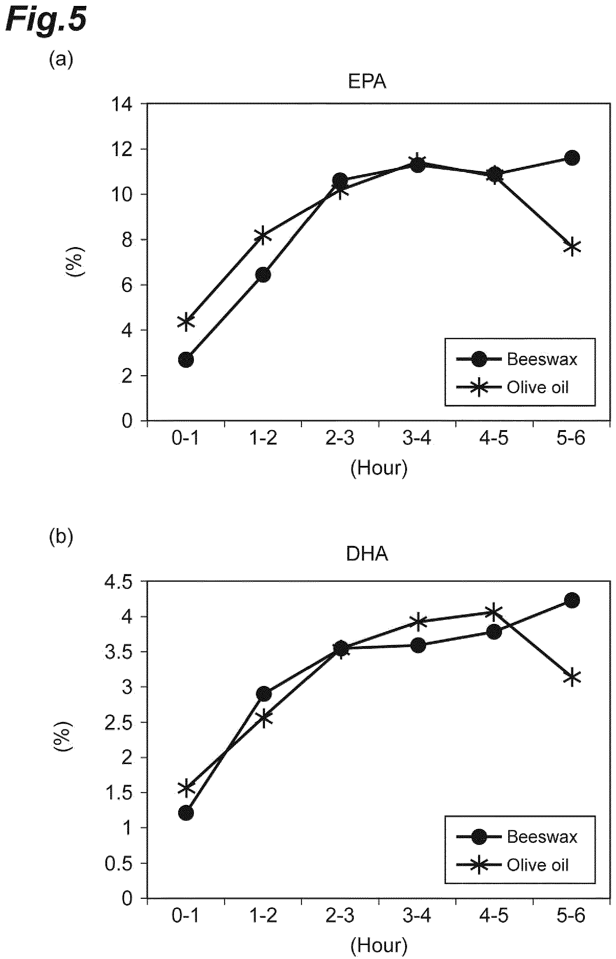

Figure 5 shows the EPA and DHA concentrations in the lymph fluid 0-1 hours, 1-2 hours, 2-3 hours, 3-4 hours, 4-5 hours and 5-6 hours after gavage administration of the sample. Figure 5 (a) shows the concentration of EPA and Figure 5 (b) shows the concentration of DHA. The EPA and DHA concentrations in the lymph fluid increased in both of the group to which the olive oil was added and the group to which the beeswax was added over a period of 1 to 5 hours after administration. The EPA and DHA concentrations in the lymph fluid reduced in the group to which the olive oil was added over a period of 5 to 6 hours after administration. On the other hand, the reduction of the EPA and DHA concentrations was not detected in the group to which the beeswax was added.

### [Test Example II]

The unsaturated fatty acid absorption accelerating effect of the beeswax on human was examined. Five subjects were selected who were healthy adult males, aged 20 to 40 with a BMI (Body Mass Index) value of 18.5 or more and below 25.0, substantially had no chance to regularly ingest fish and were able to refrain from ingesting fish during the test period and passed the screening test in advance.

A soft capsule containing 20 mg of EPA, 80 mg of DHA and 5 mg of the beeswax per capsule was used as the test food product. Additionally, a soft capsule containing 20 mg of EPA and 80 mg of DHA per capsule and produced in the same manner as the test food product, except that the beeswax was not contained, was used as the control food product.

The subjects were fasted for 10 hours or more and allowed to ingest 5 capsules of the above test food products with 150 mL of water (Phase I ingestion). Immediately before ingestion of the test food product and 6 hours after ingestion, 5 mL each of blood was collected from the forearm vein of each of the subjects using a heparin sodium containing vacuum blood collection tube. The collected blood samples were subjected to a refrigerated centrifuge (4°C, 3000 rpm x 10 min), subsequently the plasma was separately collected and cryopreserved at -20°C until measurement.

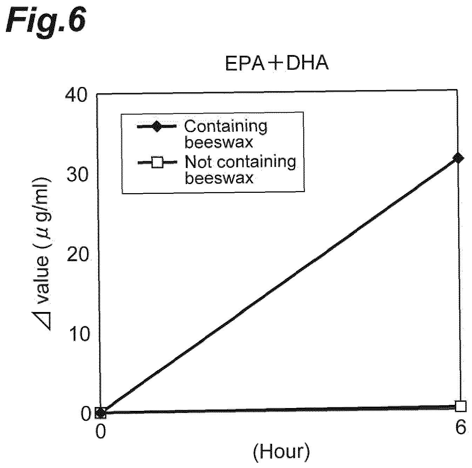

The total lipid obtained from the plasma by the Folch method was methyl-esterified by hydrochloric acid-methanol method and subsequently subjected to gas chromatography to measure the EPA and DHA concentrations in the plasma. Table 2 shows amounts of changes (µg/mL) of the EPA and DHA concentrations in the plasma of each subject at 6 hours after ingestion from immediately before ingestion. Figure 6 shows the average value of the five subjects.

After a washout period of 7 days or more from the day of Phase I ingestion, Phase II ingestion was conducted to the same subjects by the same method as for Phase I ingestion, with the exception that the test food product was replaced with the control food product. The EPA and DHA concentrations in the plasma were measured by the same method as above. Table 2 shows the amounts of changes (µg/mL) of the EPA and DHA concentrations in the plasma of each subject at 6 hours after ingestion from immediately before ingestion. Figure 6 shows the average value of the five subjects.

**[Table 2]**

| | | Subjects | | | | | Average value | Standard deviation | |
|---|---|---|---|---|---|---|---|---|---|
| | | A | B | C | D | E | | | |
| Plasma EPA+DHA concentration Δ value (µg/ml) | Test food product (containing beeswax) | 59.9 | 18.3 | 13.5 | 44.4 | 21.0 | 31.4 | 19.875 | p<0.05 |
| | Control food product (not containing beeswax) | 6.1 | -5.0 | -0.2 | 5.1 | -4.5 | 0.3 | 5.215 | |

It is revealed that, when the test food product containing the beeswax in addition to EPA and DHA was ingested, the total amount of EPA and DHA concentrations in the plasma after ingestion was higher than the case where the control food product not containing the beeswax was ingested.

## Claims

1. An unsaturated fatty acid absorption accelerator comprising a wax as an active ingredient.

2. The absorption accelerator according to Claim 1, wherein the wax is a beeswax.

3. The absorption accelerator according to Claim 1 or 2, wherein the unsaturated fatty acid is one or more selected from the group consisting of n-3 polyunsaturated fatty acids and n-6 polyunsaturated fatty acids.

4. The absorption accelerator according to any one of Claims 1 to 3, wherein the unsaturated fatty acid is at least one selected from the group consisting of eicosapentaenoic acid and docosahexaenoic acid.

5. A capsule comprising a capsule film and a composition encapsulated inside the capsule film, wherein the composition comprises a wax and an unsaturated fatty acid.

6. A capsule comprising a capsule film and a composition encapsulated inside the capsule film, wherein the capsule film comprises a wax and wherein the composition comprises an unsaturated fatty acid.

7. The capsule according to Claim 5 or 6, wherein the wax is a beeswax.

8. The capsule according to any one of Claims 5 to 7, wherein the unsaturated fatty acid is one or more selected from the group consisting of n-3 polyunsaturated fatty acids and n-6 polyunsaturated fatty acids.

9. The capsule according to any one of Claims 5 to 8, wherein the unsaturated fatty acid is at least one selected from the group consisting of eicosapentaenoic acid and docosahexaenoic acid.
